(19) Europäisches Patentamt
European Patent Office
Office européen des brevets

(11) Numéro de publication: 0 064 006
B1

(12) # FASCICULE DE BREVET EUROPEEN

(45) Date de publication du fascicule du brevet: **31.10.84**

(51) Int. Cl.³: **C 07 C 143/70**, C 07 C 143/79, C 07 C 143/78, C 07 D 295/12

(21) Numéro de dépôt: **82400718.1**

(22) Date de dépôt: **21.04.82**

(54) **Chlorures de dialcoxy (2,4) benzènesulfonyle substitués.**

(30) Priorité: **23.04.81 FR 8108126**

(43) Date de publication de la demande: **03.11.82 Bulletin 82/44**

(45) Mention de la délivrance du brevet: **31.10.84 Bulletin 84/44**

(84) Etats contractants désignés: **AT BE CH DE GB IT LI LU NL SE**

(56) Documents cités:
**FR - A - 2 338 929**
**FR - A - 2 378 004**

**JOURNAL OF MEDICINAL CHEMISTRY, vol. 20, no. 10, octobre 1977, P. JACOB et al.: "Monomethylthio analogues of 1-(2,4,5-Trimethoxyphenyl)-2-aminopropane", pages 1235-1239**
**JOURNAL OF THE CHEMICAL SOCIETY, Perkin I, 1978, F. BOTTINI et al.: "Synthesis of two novel (2.2) Metacyclophanes, 4,6,12,14-tetramethyl-and 4,6,12, 14-tetramethoxy-1,2,9,10-tetrathia (2.2)metacyclophane", pages 198-200**

(73) Titulaire: **CHOAY S.A., 48, Avenue Théophile-Gautier, F-75782 Paris Cédex 16 (FR)**

(72) Inventeur: **Fournier, Jean-Paul, 10, rue Fontenay, F-78000 Versailles (FR)**
Inventeur: **Choay, Patrick, 7, Cours Jasmin, F-75016 Paris (FR)**

(74) Mandataire: **Gutmann, Ernest et al, Cabinet Plasseraud 84, rue d'Amsterdam, F-75009 Paris (FR)**

EP 0 064 006 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention est relative à de nouveaux composés du type sulfohalogénures trisubstitués ainsi qu'à leur procédé de préparation.

L'invention vise également l'application de ces nouveaux sulfohalogénures, en particulier dans la préparation des nouveaux composés du type benzènesulfonamides trisubstitués utiles notamment à titre de principe actif de nouveaux médicaments.

Les sulfohalogénures trisubstitués selon l'invention sont des benzènesulfohalogénures substitués en position 2, 4, 5, et répondant à la formule suivante:

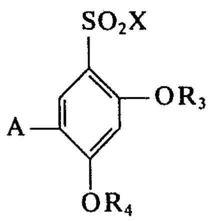

(I)

dans laquelle:

| | |
|---|---|
| X | est un atome d'halogène, notamment le brome ou de préférence le chlore, |
| $R_3$ et $R_4$ | représentent chacun, indépendamment l'un de l'autre, un radical alcoyle inférieur ayant de 1 à 4 atomes de carbone, |
| A | représente un halogène, les radicaux alcoxy ayant de 1 à 4 atomes de carbone, les groupes alcoylsulfonyle ayant de 1 à 4 atomes de carbone, le groupe $NO_2$, $CF_3$. |

Une classe préférée de sulfohalogénures conformes à l'invention est constituée par des dialcoxy (2, 4) benzènesulfochlorures substitués en position 5 et répondant à la formule (II) suivante:

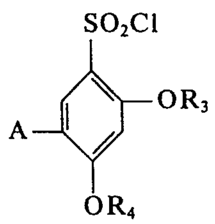

(II)

dans laquelle A, $R_3$ et $R_4$ ont les significations ci-dessus indiquées.

Une classe préférée de sulfohalogénures conformes à l'invention, est constituée par les diméthoxy (2, 4) benzènesulfohalogénures substitués en position 5 et répondant à la formule:

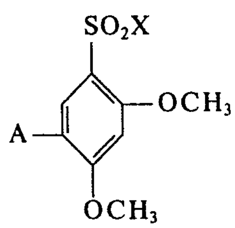

(III)

dans laquelle A et X ont les significations ci-dessus indiquées.

Une classe préférée de benzènesulfochlorures trisubstitués conformes à l'invention est constituée par les sulfochlorures de formule (IV) suivante:

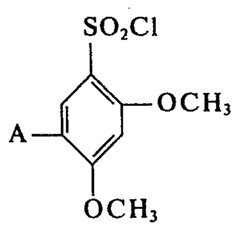

(IV)

dans laquelle A a la significations ci-dessus indiquée.

Une autre classe préférée de sulfochlorures conformes à l'invention est constituée par ceux de formule (IV) dans laquelle A représente avantageusement l'un des radicaux suivants: Cl, Br, $OCH_3$, $SO_2CH_3$, $SO_2C_2H_5$, $SO_2nC_3H_7$, $SO_2iC_3H_7$, de préférence Cl, Br, $OCH_3$.

Pour préparer les sulfohalogénures de formule (I):

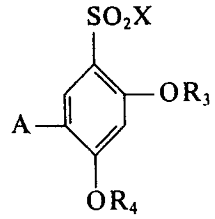

(I)

dans laquelle X, A, $R_3$ et $R_4$ ont les significations ci-dessus indiquées, on peut avoir recours à l'arylamine de formule (V):

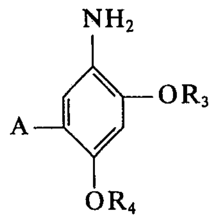

(V)

dans laquelle A, $R_4$ et $R_5$ ont les significations indiquées ci-dessus, et à partir de laquelle:

a) on forme le sel de diazonium, notamment le chlorure de diazonium de formule (VI). Ce sel de diazonium est notamment obtenu en faisant réagir l'arylamine au sein d'une solution de l'acide halogéné correspondant, notamment d'acide chlorhydrique, avec une solution de nitrite d'un métal alcalin, et en maintenant le mélange réactionnel à une température de préférence inférieure à environ 10°C;

b) on fait ensuite réagir le sel de diazonium obtenu en solution avec de l'anhydride sulfureux. On opère de préférence en présence d'acide acétique, ainsi que d'un catalyseur apte à contribuer à la transformation du groupe diazonium en groupe sulfohalogénure, notamment sulfochlorure. Ce catalyseur est, par exemple à base de cuivre (réaction de Sandmeyer modifiée).

Les étapes a) et b) de cette réaction (appliquées à l'obtention des sulfochlorures trisubstitués de formule (II) peuvent être représentées comme suit:

(V) 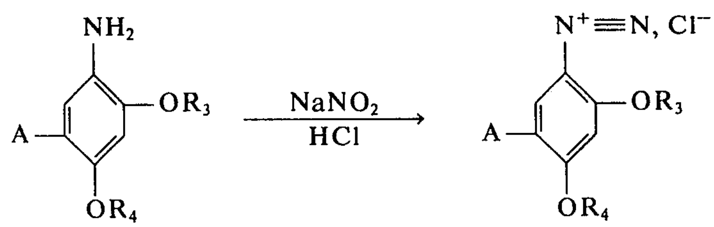

(VI)

(VI) 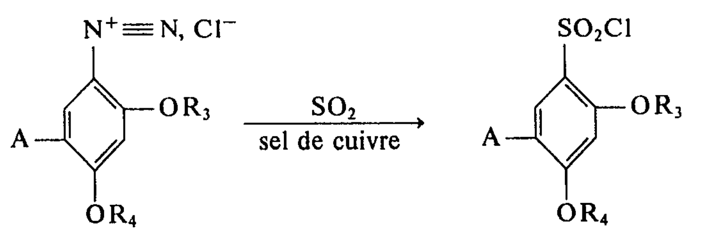

(II)

Ce schéma réactionnel s'applique également pour la préparation des sulfohalogénures de formule (I).

A titre d'arylamines préférées pour la préparation des sulfochlorures de formule (II) on peut citer:

- la chloro-5 diméthoxy-2,4 aniline,
- la bromo-5 diméthoxy-2,4 aniline,
- la triméthoxy-2,4,5 aniline,
- la diméthoxy-2,4 méthylsulfonyl-5 aniline,
- la diméthoxy-2,4 éthylsulfonyl-5 aniline,
- la diméthoxy-2,4 propylsulfonyl-5 aniline,
- la diméthoxy-2,4 isopropylsulfonyl-5 aniline.

Pour préparer les sulfohalogénures de formule (I), notamment les sulfochlorures de formule (II), dans laquelle A, $R_3$ et $R_4$ ont la signification ci-dessus indiquée, on peut également procéder par sulfonation

ou halogénosulfonation, de préférence chlorosulfonation du composé de formule (VII) dans laquelle A, $R_3$ et $R_4$ ont les significations susindiquées:

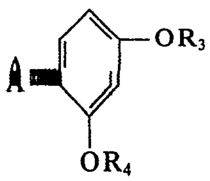

(VII)

Dans le cas de la sulfonation — par réaction du composé (VII) — avec de l'acide sulfurique, on obtient dans une première étape, l'acide sulfonique de formule (VIII):

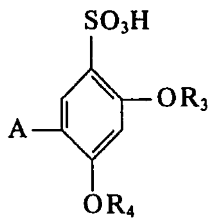

(VIII)

L'acide de formule (VIII) obtenu peut ensuite être transformé en sel d'une base organique ou minérale, par action de la base appropriée telle que l'hydroxyde de sodium, l'hydroxyde de potassium, ou la pyridine.

Ce sel est représenté par la formule (IX) suivante:

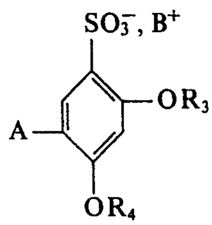

(IX)

dans laquelle $B^+$ représente un métal, notamment alcalin ou alcalino-terreux, et A a l'une quelconque des significations indiquées ci-dessus. L'halogénure de formule (I) et notamment le chlorure de formule (II) est alors obtenu par action sur le composé de formule (VIII) — ou sur un sel organique ou minéral correspondant de formule (IX) — d'un agent halogénant, notamment chlorant, tel que le chlorure de thionyle, le pentachlorure de phosphore, ou l'oxychlorure de phosphore.

A titre d'exemple, le schéma réactionnel de l'obtention des composés de formule (II) dans lesquels A, $R_3$ et $R_4$ ont l'une quelconque des significations indiquées ci-dessus, à partir des composés de formule (VII) peut se représenter comme suit:

(VII)

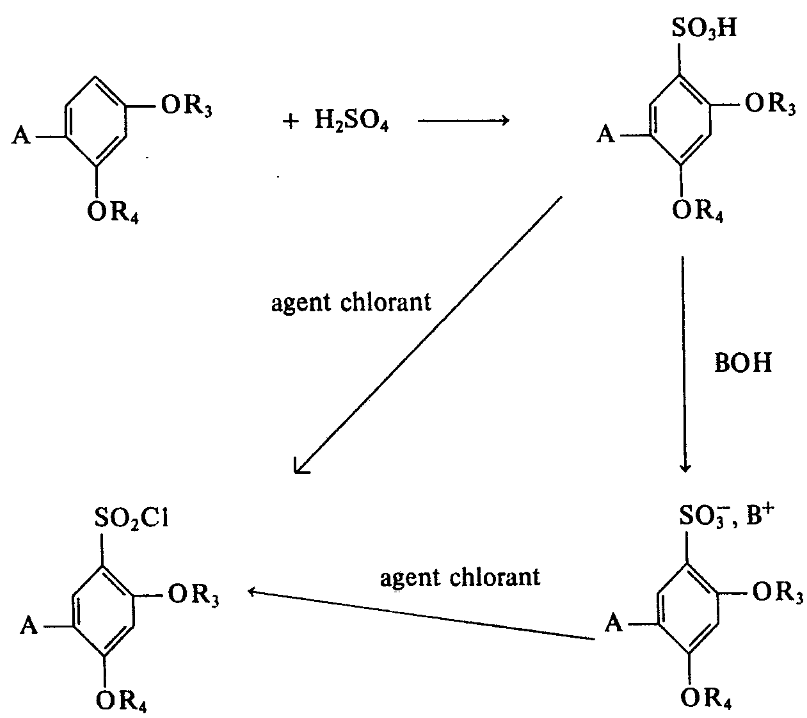


(VIII)

(II)

(IX)

Ce schéma réactionnel s'applique également pour la préparation des sulfohalogénures de formule (I).

Dans le cas de l'halogénosulfonation, notamment la chlorosulfonation, on fait réagir l'acide halogénosulfonique, notamment chlorosulfonique sur le composé de formule (VII) dans laquelle A, $R_3$ et $R_4$ ont les significations ci-dessus indiquées.

La réaction appliquée à titre d'exemple, à l'obtention des sulfochlorures de formule (II) peut s'écrire:

(VII) 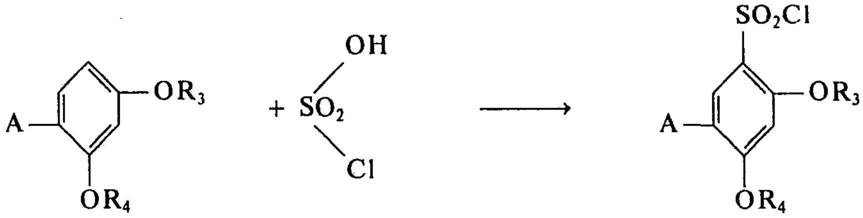 (II)

Ce schéma réactionnel s'applique également pour la préparation des sulfohalogénures de formule (I).

Les nouveaux sulfohalogénures conformes à l'invention, et plus particulièrement les nouveaux sulfochlorures, sont utiles notamment à titre de produits intermédiaires pour la préparation des nouveaux dialcoxy (2,4) benzènesulfonamides substitués en position 5 de formule (X):

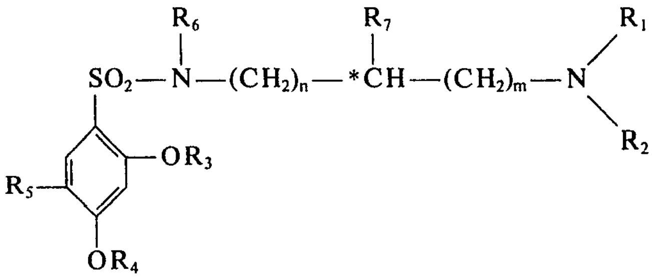

(X)

dans laquelle:

n et m — indépendamment l'un de l'autre prennent les valeurs de 0 à 4;

$R_1$ et $R_2$ — représentent indépendamment l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substituése ou non par des radicaux alcoyle linéaires ou ramifiés ayant de 1 à 4 atomes de carbone;

$R_3$ et $R_4$ — ont les mêmes significations que celles indiquées dans la définition de la formule (I) c'est-à-dire représentent chacun, indépendamment l'un de l'autre, un radical alcoyle inférieur présentant de 1 à 4 atomes de carbone;

$R_5$ — a la même signification que celle de A indiquée dans la définition de la formule (I), c'est-à-dire représente: un halogène, le groupe $NO_2$, $CF_3$, un radical alcoxy ayant de 1 à 4 atomes de carbone, ou un radical alcoylsulfonyle ayant de 1 à 4 atomes de carbone et peut également représenter le groupe $NH_2$;

$R_6$ et $R_7$ — représentent indépendamment l'un de l'autre un atome d'hydrogène, un radical alcoyle de 1 à 6 atomes de carbone ou un radical cycloalcoyle de 3 à 6 atomes de carbone.

Les nouveaux sulfohalogénures conformes à l'invention, notamment les sulfochlorures, sont également utiles pour la préparation des isomères optiques des composés de formule (X), lorsque $R_7$ est différent de l'hydrogène.

Les nouveaux sulfohalogénures conformes à l'invention, notamment les sulfochlorures, sont également utiles pour la préparation des sels physiologiquement acceptables des composés de formule (X), obtenus avec des acides organiques ou minéraux.

Une classe préférée de nouveaux dialcoxy (2,4) benzènesulfonamides substitués en position 5 préparés à partir des sulfohalogénures, notamment des sulfochlorures conformes à l'invention, est constituée par les composés de formule:

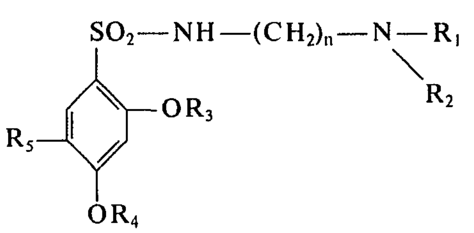

(XI)

dans laquelle n, $R_1$, $R_2$, $R_3$, $R_4$ et $R_5$ ont les significations indiquées ci-dessus.

Une classe préférée de nouveaux dialcoxy (2,4) benzènesulfonamides substitués en position 5 préparés à partir des sulfohalogénures, notamment des sulfochlorures conformes à l'invention, est constituée par les composés de formule:

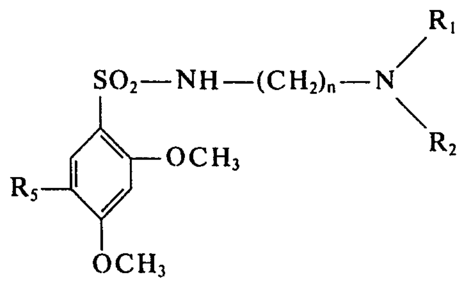

(XII)

dans laquelle n, $R_1$, $R_2$ et $R_5$ ont les significations indiquées ci-dessus.

Une classe préférée de nouveaux dialcoxy (2,4) benzènesulfonamides substitués en position 5 préparés à partir des sulfohalogénures, notamment des sulfochlorures conformes à l'invention, est constituée par les composés de formule (XII) dans laquelle $R_5$ représente $SO_2CH_3$, $SO_2C_2H_5$, $SO_2nC_3H_7$, $SO_2iC_3H_7$, et de préférence Cl, Br, $OCH_3$.

Les composés de ces divers dialcoxy (2,4) benzènesulfonamides sont avantageusement sous forme de sels, notamment de chlorhydrate.

Pour préparer le chlorhydrate de ces nouveaux dialcoxy (2, 4) benzènesulfonamides substitués en position 5 on peut faire agir une amine de formule:

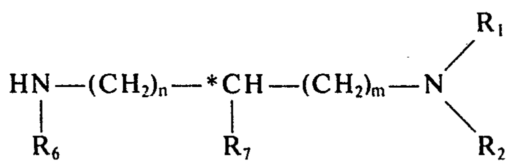

(XIII)

dans laquelle n, m, $R_1$, $R_2$, $R_6$ et $R_7$ ont les significations ci-dessus indiquées sur un sulfochlorure conforme à l'invention, de formule (II):

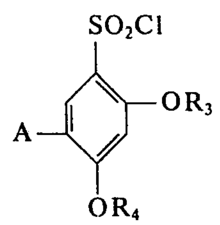

(II)

dans laquelle $R_3$ et $R_4$ ont les significations sus-indiquées et A a l'une quelconque des significations indiquées ci-dessus pour $R_5$ à l'exception du groupe $NH_2$ (étant etendu que l'on obtiendra un autre halogénate des composés de formule (X), si l'on met en oeuvre un autre sulfohalogénure, par exemple un sulfobromure, en lieu et place du susdit sulfochlorure).

L'obtention des composés de formule (X) dans laquelle $R_5$ vaut $NH_2$ est réalisée en réduisant le composé de formule (X) dans lequel $R_5$ vaut $NO_2$ par hydrogénation catalytique ou par réduction chimique.

On obtient ainsi les chlorhydrates de formule ci-après:

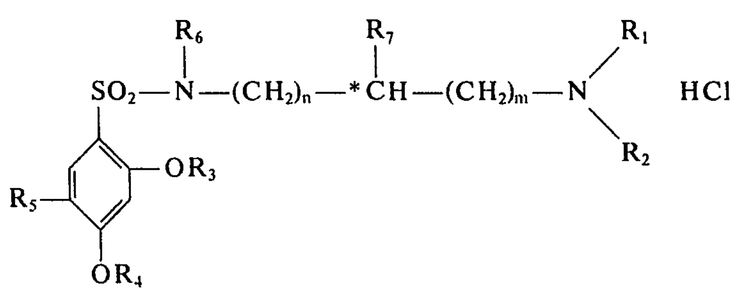

(XIV)

Le passage du chlorhydrate de formule (XIV) au composé de formule (X), non salifié, c'est-à-dire sous forme de base, peut se faire en solution, par réaction avec une base forte telle que l'hydroxyde de sodium ou de potassium, ou toute autre base présentant des caractéristiques équivalentes.

La transformation du chlorhydrate de formule (XIV) en un sel différent peut se faire en passant par la base de formule (X), puis en salifiant celle-ci selon des procédés classiques.

Un groupe d'amines préférées utilisées dans la préparation des composés de formule (X) ou de leurs sels correspondants, est constitué par les suivantes:

— la diméthylamino-éthylamine,
— la diéthylamino-éthylamine,
— la pyrrolidino-éthylamine,
— la pipéridino-éthylamine,
— la morpholino-éthylamine,
— la diméthylamino-propylamine,
— la diéthylamino-propylamine,
— la pipéridino-propylamine,
— la (méthyl-2 pipéridino)-3 propylamine,
— la morpholino-propylamine.

Les exemples suivants relatifs à la préparation d'un certain nombre de nouveaux sulfochlorures et de nouveaux dialcoxy (2,4) benzènesulfonamides substitués en position 5 servent à illustrer l'invention, mais ne sont pas limitatifs.

Exemple 1

Preparation du chlorure de Trimethoxy-2,4,5 benzenesulfonyle

Elle peut être effectuée selon deux procédés:

1er procédé

Dans un tricol de 500 $cm^3$, muni d'un thermomètre, d'une ampoule à réactif, d'une garde à chlorure de calcium et d'un système d'agitation magnétique, sont placés 42 g (0,25 mole) de triméthoxy-1,2,4 benzène en solution dans 200 $cm^3$ de chloroforme pur. Au milieu réactionnel, placé sous atmosphère d'azote et refroidi vers −10°C, sont ajoutés goutte à goutte 80 $cm^3$ d'acide chlorosulfonique. Il se forme successivement un précipité laiteux blanc-crème, puis une solution verdâtre qui vire au brun. L'addition terminée, le milieu réactionnel est laissé en contact 1 h à température ambiante, puis est versé sur de la glace pilée. Le précipité obtenu est extrait par du chloroforme; la phase organique est ensuite séchée sur sulfate de sodium puis évaporée sous pression réduite. Le résidu brun formé est lavé avec le minimum de toluène jusqu'à l'obtention d'un solide beige qui est ensuite chromatographié sur colonne de silice.
L'élution avec du benzène, puis avec un mélange benzène-chloroforme (50—50) donne le chlorure de triméthoxy-2,4,5 benzènesulfonyle.

Rdt 46%, F 147°C
RMN ($CDCl_3$) à 80 MHz:
$\delta$ 7,29 ppm (s 1 H ArH); $\delta$ 6,55 ppm (s 1 H ArH); $\delta$ 3,93, 3,78 et 3,53 ppm (3 s 9 H $OCH_3$)
IR (KBr) $\nu$ $SO_2$, as 1350 $cm^{-1}$, s 1160 $cm^{-1}$.

2ème procédé

Il comporte 4 étapes

Diméthoxy-3,4 chlorobenzène

Dans un tricol d'un litre, muni d'un système d'agitation magnétique, d'un thermomètre, d'une garde à chlorure de calcium et d'une ampoule à réactif, sont introduits à froid vers 0°C et successivement 138 g (1 mole) de vératrole puis goutte à goutte 135 g (1 mole) de chlorure de sulfuryle. L'addition terminée, le milieu réactionnel est ramené à température ambiante, puis après contact une heure est distillé sous pression réduite.

Rdt 83%, PE: 120°C sous 1 999,83 Pa (15 mm Hg)

Diméthoxy-4,5 nitro-2 chlorobenzène

Dans un tricol d'un litre, muni d'un système d'agitation magnétique, d'un thermomètre et d'une ampoule à réactif, sont introduits successivement 143,9 g (0,83 mole) de diméthoxy-3,4 chlorobenzène, puis goutte à goutte 166 $cm^3$ d'acide nitrique (d = 1,38), sans que la température dépasse 25°C.

L'addition terminée, le mélange réactionnel est laissé 1,5 h en contact puis filtré.

Rdt 95%, F 105°C

Triméthoxy-2,4,5 nitrobenzène

Dans un ballon de 2 litres, muni d'un réfrigérant, sont introduits successivement une solution de potasse méthanolique (100 g de KOH dans 500 $cm^3$ de méthanol), puis 100 g (0,46 mole) de diméthoxy-4,5 nitro-2 chlorobenzène et du carborandum. Le mélange est porté à l'ébullition au reflux 6 h. Après refroidissement, le milieu réactionnel est filtré; le précipité obtenu est lavé avec du méthanol.

Rdt 95%, F 129°C

Triméthoxy-2,4,5 aniline

Dans un ballon de 500 $cm^3$, muni d'un réfrigérant, sont ajoutés successivement 21,3 g (0,1 mole) de triméthoxy-2,4,5 nitrobenzène, 80 g de chlorure stanneux chimiquement pur pour miroitiers, 100 $cm^3$ d'une solution d'acide chlorhydrique (d = 1,18) et du carborandum. Le mélange est porté à l'ébullition à reflux 1 h. Après refroidissement, une solution de lessive de soude est ajoutée jusqu'à dissolution du précipité. La solution obtenue est extraite par du chlorure de méthylène. Les extraits organiques sont séchés sur sulfate de sodium puis évaporés sous pression réduite. Le résidu obtenu est cristallisé dans de l'éthanol.

Rdt 80%, F 94°C

Chlorure de triméthoxy-2,4,5 benzènesulfonyle

Dans un tricol de 250 $cm^3$, muni d'un système d'agitation et d'un thermomètre, sont introduits successivement 18,3 g (0,1 mole de triméthoxy-2,4,5 anilline puis 50 $cm^3$ d'une solution d'acide chlorhydrique (d = 1,18). Après un contact de 4 heures, l'amine est diazotée à −5°C par addition d'une solution de nitrite de sodium (10 g de $NaNO_2$ dans 50 $cm^3$ d'eau). Le sel de diazonium obtenu est versé lentement dans un tricol, chauffé à 40°C et so us atmosphère d'azote, contenant 200 $cm^3$ d'acide acétique saturé en anhydride sulfureux et 7 g de chlorure cuivrique. Le mélange est porté 2 h à 60°C, puis est versé sur de la glace pilée.

Rdt 35%, F 147°C

Exemple 2

Préparation du chlorure de chloro-5 diméthoxy-2,4 benzènesulfonyle

Elle est effectuée en 2 étapes à partir du diméthoxy-1,3 benzène:

1ère étape

Obtention du diméthoxy-2,4 chlorobenzène

Elle s'effectue en suivant la technique de G. CASTELFRANCHI et G. BORRA rapportée dans Annali di Chimica, 1953, 43, 293.

Dans un tricol de 500 $cm^3$, muni d'un système d'agitation magnétique, d'un thermomètre, d'une garde à chlorure de calcium et d'une ampoule à réactif, et refroidi vers 10°C, sont introduits successivement 97,5 g (0,70 mole) de diméthoxy-1,3 benzène puis goutte à goutte 96,5 g (0,70 mole) de chlorure de sulfuryle. Une fois l'addition terminée, la solution est ramenée à la température ambiante et laissée en contact 2 h puis distillée.

Rdt 85%, PE: 137°C sous 2 399,80 Pa (18 mm Hg)

2ème étape

Passage au chlorure de chloro-5 diméthoxy-2,4 benzènesulfonyle

Dans un tricol de 500 $cm^3$, muni d'un système d'agitation magnétique, d'une garde à chlorure de calcium, d'un thermomètre et d'une ampoule à réactif, sont introduits 35 g (0,2 mole) de diméthoxy-2,4 chlorobenzène en solution dans 250 $cm^3$ de chloroforme pur. La solution est refroidie vers 0 °C, puis est additionnée goutte à goutte de 50 $cm^3$ (0,75 mole) d'acide chlorosulfonique. Une fois l'addition terminée, le milieu réactionnel est ramené à température ambiante, puis laissé en contact 3 h; il est ensuite versé sur de la glace pilée. Le mélange obtenu est épuisé au chloroforme. Les extraits organiques sont séchés sur sulfate de sodium puis concentrés jusqu'à cristallisation. Le solide obtenu est recristallisé dans un mélange éther éthylique/benzène.

Rdt 74%, F 175 °C
RMN ($CDCl_3$) à 80 MHz:
$\delta$ 7,87 ppm (s 1 H ArH); $\delta$ 6,55 ppm (s 1 H ArH); $\delta$ 4 et 3,96 ppm (2 s 6 H $OCH_3$)
IR (KBr): $\nu SO_2$, as 1385 $cm^{-1}$, s 1170 $cm^{-1}$

Exemple 3

Préparation du chlorure de bromo-5 diméthoxy-2,4 benzènesulfonyle

Elle est effectuée en 2 étapes à partir du diméthoxy-1,3 benzène:

1ère étape

Obtention du diméthoxy-2,4 bromobenzène

Elle s'effectue en suivant le protocole de S. T. FENG et K. Y. CHIU rapportée dans Hsûeh Pao, 1959, 25, 277.
Dans un tricol de 250 $cm^3$, muni d'un système d'agitation magnétique, d'un thermomètre, et refroidi vers 10°C, sont ajoutés successivement 27,6 g (0,2 mole) de diméthoxy-1,3 benzène puis par petites portions 36 g (0,2 mole) de N-bromosuccinimide. Une fois l'addition terminée, le milieu réactionnel est ramené à température ambiante et laissé en contact 2 h. Après lavage à l'eau et extraction au chloroforme, les extraits organiques sont séchés sur sulfate de sodium puis concentrés sous pression réduite. Le liquide résiduel est distillé.

Rdt 82%, PE: 150°C sous 1 999,83 Pa (15 mm Hg)

2ème étape

Passage au chlorure de bromo-5 diméthoxy-2,4 benzènesulfonyle

Il est obtenu selon le même protocole que celui décrit dans l'exemple 3 pour préparer le chlorure de chloro-5 diméthoxy-2,4 benzènesulfonyle. En utilisant 65,1 g (0,3 mole) de diméthoxy-2,4 bromobenzène et 100 $cm^3$ d'acide chlorosulfonique, le rendement est de 77%.

F 195°C
RMN ($CDCl_3$) à 60 MHz
$\delta$ 7,85 ppm (s 1 H ArH); $\delta$ 6,70 ppm (s 1 H ArH); $\delta$ 3,92 et 3,88 ppm (2 s 6 H $OCH_3$);
IR (KBr); $\nu SO_2$, as 1385 $cm^{-1}$, s 1165 $cm^{-1}$

Exemple 4

Chlorures d'alkylsulfonyl-5 diméthoxy-2,4 benzènesulfonyles

Ils sont obtenus à partir du chlorure de diméthoxy-2,4 benzènesulfonyle dont la préparation est rapportée par C. M. SUTER et H. L. HANSEN dans J. of Am. Chem. Soc. 1933, 55, 2080. Le schéma général de leur préparation est le suivant:

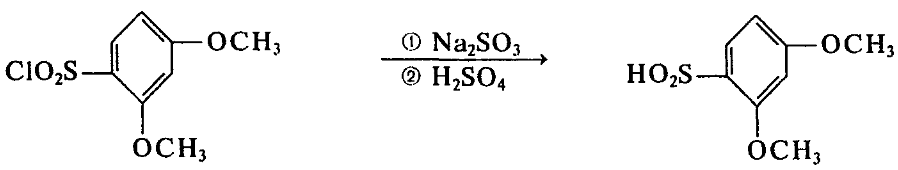


Chlorure de diméthoxy-2,4 benzènesulfonyle

Acide diméthoxy-2,4 benzènesulfinique

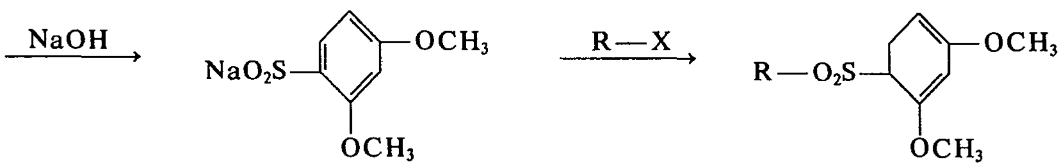


Diméthoxy-2,4 benzène-sulfinate de sodium

(Diméthoxy-2,4 phényl) alkylsulfone

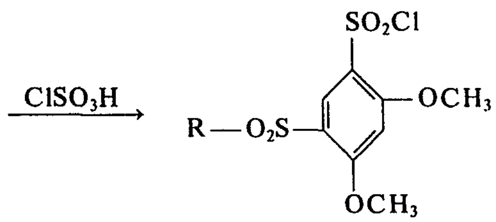


Chlorure d'alkylsulfonyl-5 diméthoxy-2,4 benzènesulfonyle

### Acide diméthoxy-2,4 benzènesulfinique

A une solution aqueuse contenant 129 g (0,974 mole) de sulfite de sodium sont ajoutés par petites portions et sous agitation 115 g (0,487 mole) de chlorure de diméthoxy-2,4 benzènesulfonyle. Durant cette opération, le pH est maintenu alcalin par addition de lessive de soude. Après 3 h de contact, le milieu réactionnel est filtré; le filtrat est acidifié par de l'acide sulfurique 2 N jusqu'à précipitation de l'acide sulfinique.

Rdt 72%, F 122°C

### Diméthoxy-2,4 benzènesulfinate de sodium

Il est obtenu par addition de la quantité stoechiométrique d'hydroxyde de sodium en solution dans l'eau. La solution de sulfinate est évaporée à sec.

### (Diméthoxy-2,4 phényl) alkylsulfones

#### Protocole général

Dans un ballon de 500 $cm^3$, muni d'un réfrigérant et d'un système d'agitation magnétique, sont ajoutés successivement 0,1 mole de diméthoxy-2,4 benzènesulfinate de sodium, 250 $cm^3$ d'isopropanol, puis 0,15 mole d'halogénure d'alkyle, de préférence un iodure. Le mélange est porté à l'ébullition à reflux 5 à 30 h selon l'halogénure mis en oeuvre. Après refroidissement, le milieu réactionnel est évaporé à sec. Le résidu est repris par de l'eau puis est extrait par du chloroforme. L'évaporation de la phase organique, après séchage sur sulfate de sodium fournit une huile qui est cristallisée dans du benzène.

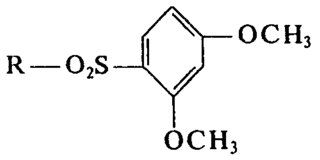

| R | PM | Temps de chauffage | F°C | Rdt % |
|---|---|---|---|---|
| $CH_3$ | 216 | 5 h | 109 | 65 |
| $C_2H_5$ | 230 | 7 h | 94 | 70 |
| $_nC_3H_7$ | 244 | 15 h | 70 | 90 |
| $_iC_3H_7$ | 244 | 30 h | 100 | 64 |

Chlorures d'alkylsulfonyl-5 diméthoxy-2,4 benzènesulfonyles

Protocole général

Dans un tricol d'un litre, muni d'un système d'agitation magnétique, d'une garde à chlorure de calcium, d'un thermomètre et d'une ampoule à réactif, est introduite une solution de (diméthoxy-2,4 phényl) alkylsulfone (0,2 mole) dans 200 $cm^3$ de chloroforme pur. Après refroidissement vers −10°C, sont ajoutés goutte à goutte 100 $cm^3$ d'acide chlorosulfonique. Une fois l'addition terminée, le mélange est laissé en contact 0,5 h à −10°C, puis est ramené à température ambiante et laissé sous agitation pendant 7 h; il est ensuite versé sur de la glace pilée. Le mélange obtenu est extrait par du chloroforme. La phase organique est séchée sur sulfate de sodium, filtrée, puis évaporée sous pression réduite. Le résidu est cristallisé dans du benzène.

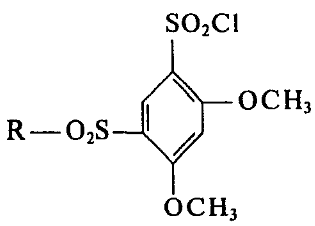

| R | PM | F°C | Rdt % |
|---|---|---|---|
| $CH_3$ | 314,5 | 204 | 52 |
| $C_2H_5$ | 328,5 | 191 | 77 |
| $_nC_3H_7$ | 342,5 | 169 | 87 |
| $_iC_3H_7$ | 342,5 | 218 | 66 |

Chlorure de diméthoxy-2,4 méthylsulfonyl-5 benzènesulfonyle

RMN (DMSO) à 80 MHz:
$\delta$ 8,2 ppm (s 1 H ArH); $\delta$ 6,9 ppm (s 1 H ArH); $\delta$ 4,1 ppm et 4,05 ppm (2 s 6 H $OCH_3$); 3,2 ppm (s 3 H $CH_3$).
IR (KBr):
$\nu$ $SO_2-Cl$, as 1360 $cm^{-1}$, s 1170 $cm^{-1}$;
$\nu$ $SO_2-CH_3$, as 1300 $cm^{-1}$, s 1140 $cm^{-1}$.

### Chlorure d'éthylsulfonyl-5 diméthoxy-2,4 benzènesulfonyle

RMN (DMSO) à 80 MHz:
$\delta$8,2 ppm (s 1 H ArH); $\delta$6,93 ppm (s 1 H ArH); $\delta$4,15 ppm et 4,1 ppm (2 s 6 H $OCH_3$); $\delta$3,25 ppm (q 2 H $-CH_2-$); $\delta$ 1,18 ppm (t 3 H $CH_3-$).
IR (KBr):
$\nu$ $SO_2-Cl$, as 1370 $cm^{-1}$, s 1175 $cm^{-1}$;
$\nu$ $SO_2-CH_2-$, as 1315 $cm^{-1}$, s 1140 $cm^{-1}$.;

### Chlorure de diméthoxy-2,4 propylsulfonyl-5 benzènesulfonyle

RMN (DMSO) à 80 MHz:
$\delta$ 8,05 ppm (s 1 H ArH); $\delta$ 6,72 ppm (s 1 H ArH); 3,92 et 3,87 ppm (2 s 6 H $OCH_3$); $\delta$ 3,2 ppm (t 2 H$-CH_2-SO_2-$); $\delta$ 1,45 ppm (m 2 H $-CH_2-$); $\delta$ 0,85 ppm (t 3 H $CH_3$).
IR (KBr):
$\nu$ $SO_2Cl$, as 1370 $cm^{-1}$, s 1180 $cm^{-1}$;
$\nu$ $SO_2-CH_2-$, as 1315 $cm^{-1}$, s 1135 $cm^{-1}$.

### Chlorure d'isopropylsulfonyl-5 diméthoxy-2,4 benzènesulfonyle

RMN (DMSO) à 80 MHz:
$\delta$ 8,02 ppm (s 1 H ArH); $\delta$ 6,7 ppm (s 1 H ArH); $\delta$ 3,92 et 3,85 ppm (2 s 6 H $OCH_3$);

$\delta$ 3,48 ppm m 1 H $-\underset{|}{CH}-$

$\delta$ 1,11 et 1,03 ppm (2 s 6 H $CH_3$).
IR (KBr):
$\nu$ $SO_2-Cl$, as 1365 $cm^{-1}$, s 1175 $cm^{-1}$;

$\nu$ $SO_2-$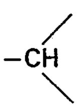

as 1300 $cm^{-1}$, s 1130 $cm^{-1}$.

## Exemple 5

### Préparation des diméthoxy-2,4 benzènesulfonamides n substitues

A 0,015 mole de chlorure de benzènesulfonyle en solution dans un mélange de chlorure de méthylène (40 $cm^3$) et de méthanol (10 $cm^3$) est ajouté 0,015 mole de dialkylaminoalkylamine en solution dans 10 $cm^3$ de chlorure de méthylène. Après un contact de 2 h, sous agitation et à température ambiante, le milieu réactionnel est évaporé à sec puis est repris par 50 $cm^3$ d'eau. La solution aqueuse est lavée par de l'éther éthylique, puis évaporée à sec. Le résidu est cristallisé dans un mélange isopropanol/méthanol. Le passage éventuel à la base s'effectue en traitant la solution aqueuse du chlorhydrate par de la soude N jusqu'à précipitation. Le précipité est extrait par du chlorure de méthylène. La solution organique est séchée puis évaporée sous pression réduite. La base obtenue est cristallisée dans un mélange éther éthylique/éther de pétrole.

D'autres composés ont été préparés de façon analogue et sont rassembles dans le tableau I suivant (le point de fusion indiqué par chaque composé est, sauf indication contraire, celui du chlorhydrate).

Tableau I

Composés de formule

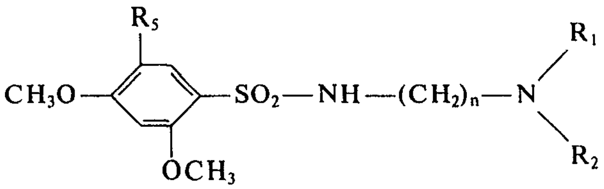

| Produit n° | 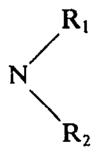 | n | $R_5$ | Formule du composé sous forme de chlorhydrate | Poids moléculaire | Point de fusion du chlorhydrate | Rendement % |
|---|---|---|---|---|---|---|---|
| 882 | 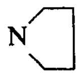 | 2 | Cl | $C_{14}H_{22}Cl_2N_2O_4S$ | 385 | 209 | 83 |
| 883 | 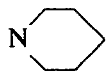 | 2 | Cl | $C_{15}H_{24}Cl_2N_2O_4S$ | 399 | 220 | 83 |
| 884 | 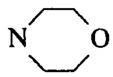 | 2 | Cl | $C_{14}H_{22}Cl_2N_2O_5S$ | 401 | 232 | 82 |
| 888 | 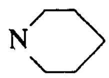 | 3 | Cl | $C_{16}H_{26}Cl_2N_2O_4S$ | 413 | 191 | 85 |
| 889 | 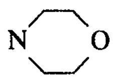 | 3 | Cl | $C_{15}H_{24}Cl_2N_2O_5S$ | 415 | 212 | 61 |
| 899 | 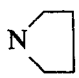 | 2 | $OCH_3$ | $C_{15}H_{25}ClN_2O_5S$ | 380,5 | 186 | 89 |
| 926 | 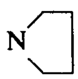 | 2 | Br | $C_{14}H_{22}BrClN_2O_4S$ | 429,75 | 226 | 64 |
| 927 | 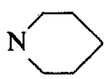 | 2 | Br | $C_{15}H_{24}BrClN_2O_4S$ | 443,78 | 204 | 71 |
| 928 | 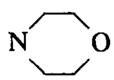 | 2 | Br | $C_{14}H_{22}BrClN_2O_5S$ | 401,75 | 246 | 71 |
| 932 | 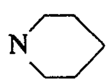 | 3 | Br | $C_{16}H_{26}BrClN_2O_4S$ | 457,80 | 200 | 60 |
| 933 | 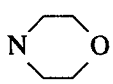 | 3 | Br | HCl,<br>$C_{15}H_{24}BrClN_2O_5S$<br>base $C_{14}H_{23}BrN_2O_5S$ | 459,77 | 237<br><br>127 | 72 |
| 957 | 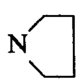 | 2 | $SO_2CH_3$ | $C_{15}H_{25}N_2O_6S_2Cl$ | 428,935 | 232 | 62 |
| 959 | 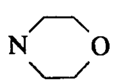 | 2 | $SO_2CH_3$ | $C_{15}H_{25}N_2O_7S_2Cl$ | 444,932 | 246 | 66 |

Fortsetzung

| Produit n° | 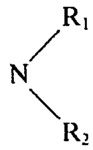 | n | $R_5$ | Formule du composé sous forme de chlorhydrate | Poids moléculaire | Point de fusion du chlorhydrate | Rendement % |
|---|---|---|---|---|---|---|---|
| 980 | 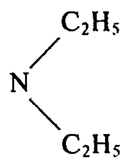 | 2 | $SO_2C_2H_5$ | $C_{16}H_{29}ClN_2O_6S_2$ | 444,978 | 242 | 71 |
| 988 | 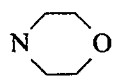 | 3 | $SO_2C_2H_5$ | $C_{17}H_{29}ClN_2O_7S_2$ | 472,986 | 204 | 70 |
| 1095 | 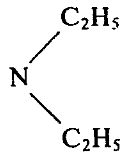 | 2 | 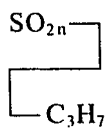 | $C_{17}H_{31}ClN_2O_6S_2$ | 459 | 224 | 91 |
| 1098 | 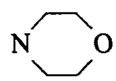 | 2 | 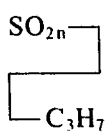 | $C_{17}H_{29}ClN_2O_7S_2$ | 472,99 | 186 | 83 |
| 1104 | 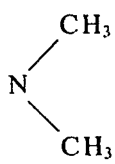 | 2 | 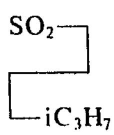 | HCl, $C_{15}H_{27}ClN_2O_6S_2$ | 430,95 | 217 | 71 |
| 1106 | 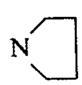 | 2 | 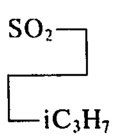 | $C_{17}H_{29}ClN_2O_6S_2$ | 456,99 | 222 | 77 |
| 1113 | 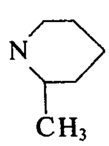 | 3 | 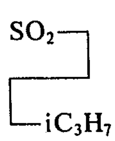 | $C_{20}H_{35}ClN_2O_6S_2$ | 499,07 | 200 | 65 |

Les nouveaux benzènesulfonamides préparés à partir des sulfochlorures selon l'invention, ainsi que leurs sels organiques ou minéraux physiologiquement acceptables présentent de remarquables propriétés pharmacologiques.

Les composés préparés à partir des sulfochlorures selon l'invention exercent une action régulatrice sur le système nerveux central et notamment une action psychomodulatrice. Ils peuvent agir notamment soit comme antidépresseurs, soit comme anxiolytiques tranquillisants.

L'activité de ces composés à partir des sulfochlorures est renforcée par le caractère lipophile de la molécule, dû à la présence d'un groupe alcoxy sur le noyau aromatique en position 4.

Les composés préparés à partir des sulfochlorures selon l'invention se distinguent aussi par le fait qu'ils potentialisent le sommeil au pentobarbital, qu'ils ne présentent pas ou peu d'affinité pour les sites dopaminergiques habituellement reconnus comme liés à certains effets indésirables (galactorrhées, aménorrhées, syndromes extrapyramidaux) et qu'ils sont dépourvus de toxicité.

Leur indice thérapeutique est compatible avec leur utilisation comme médicament.

Les composés préparés à partir des sulfochlorures selon l'invention sont avantageusement introduits comme principe actif dans le traitement des maladies à composantes dépressives, anxieuses et provoquant notamment des troubles psychosomatiques.

Les composés préparés à partir des sulfochlorures selon l'invention sont à cet effet conditionnés avec les excipients et adjuvants traditionnels, notamment ceux utilisés pour la préparation de comprimés, poudres, gélules, ampoules buvables, solutions injectables.

L'administration des médicaments contenant les composés préparés à partir des sulfochlorures selon l'invention, est effectuée de préférence par voie orale, parentérale, rectale ou locale, et les doses de composé actif administrées sont de préférence comprises entre 10 et 700 mg et notamment entre 50 et 500 mg/jour.

A titre d'exemple, différents essais pour la mise en évidence des propriétés pharmacologiques des composés préparés à partir des sulfochlorures conformes à l'invention, sont rapportés ci-après.

Essais rélatif à l'étude des interactions des médicaments selon l'invention le pentobarbital

Les essais sont réalisés sur des souris SWISS, EOPS, de sexe mâle, pesant de 20 à 24 g, en provenance du centre d'élevage de LE GENEST.

Les animaux sont acclimatés au moins 8 jours à l'animalerie du laboratoire avant les essais.

Les lots sont de 10 souris par test et par produit.

A titre de produits de référence, pour effectuer les essais témoins, on a utilisé les trois substances suivantes:

- N-[(éthyl-1 pyrrolidinyl-2) méthyl] méthoxy-2 sulfamoyl-5 benzamide connue sous la désignation sulpiride;
- N-[(éthyl-1 pyrrolidinyl-2) méthyl] méthoxy-2 éthylsulfonyl-5 benzamide connue sous la désignation sultopride;
- N-(diéthylamino-2 éthyl) méthoxy-2 méthylsulfonyl-5 benzamide connue sous la désignation tiapride.

Les composés obtenus à partir des sulfochlorures selon l'invention et les substances de référence sont administrés en suspension aqueuse dans l'eau ordinaire à 3% en gomme arabique pour la voie orale (tests d'interactions avec les barbituriques).

Le réactif pharmacologique à savoir le pentobarbital est administré en solution dans du soluté isotonique de NaCl.

Les volumes administrés sont de 0,5 ml d'une solution à 1,6 g/l pour 20 g de poids corporel par voie intrapéritonéale (40 mg/kg).

Pour effectuer ces essais, on administre à des lots de 10 souris SWISS mâles les composés obtenus à partir des sulfochlorures selon l'invention ou les produits de référence (à savoir sulpiride, sultopride ou tiapride) par voie orale à raison de 200 mg/kg. 60 minutes après l'ingestion des composés obtenus à partir des sulfochlorures selon l'invention ou des produits de référence, on administre aux animaux le pentobarbital sous forme sodique tel que celui commercialisé sous la marque NEMBUTAL. On mesure ensuite:

- le temps d'endormissement,
- le temps de sommeil,

et on détermine le pourcentage de variations en plus ou en moins du temps de sommeil. Les résultats de ces essais figurent dans le tableau II suivant.

Tableau II

| Produit n° | Temps de sommeil (pentobarbital) mn | Variation % |
|---|---|---|
| 882 | 176 ± 21 | + 144,4 |
| 883 | 176 ± 12 | + 147,9 |
| 884 | 150 ± 24 | + 111,3 |
| 888 | 140 ± 20 | + 97,2 |

Fortsetzung

| Produit n° | Temps de sommeil (pentobarbital) mn | Variation % |
|---|---|---|
| 889 | 156 ± 15 | + 119,7 |
| 899 | | + 23,1 |
| 926 | 168 ± 30 | + 133,3 |
| 927 | 169 ± 26 | + 134,7 |
| 928 | 165 ± 24 | + 129,2 |
| 932 | 142 ± 15 | + 97,2 |
| 933 | 180 ± 32 | + 150,0 |
| 957 | | + 26,8 |
| 959 | | + 41,7 |
| 980 | | + 21,1 |
| 988 | | + 9,9 |

Il est intéressant de noter que la combinaison de la substitution des positions 2,4 par des groupes alcoxy et de la substitution de la position 5 confère aux nouveaux benzènesulfonamides trisubstitués en 2,4,5 des propriétés biologiques particulièrement efficaces; cette efficacité paraît devoir être attribuée à la structure trisubstituée de la partie phényle de ces benzènesulfonamides; structure trisubstituée que l'on retrouve dans les sulfochlorures selon l'invention.

L'étude des résultats obtenus montre que les benzènesulfonamides obtenus à partir des sulfochlorures selon l'invention sont des psychomodulateurs. Ceux pour lesquels le pourcentage de la variation du temps de sommeil est supérieur à + 50% sont des potentialisateurs très nets du sommeil au pentobarbital et par conséquent peuvent être utilisés comme tranquillisants ou anxiolytiques.

Les composés obtenus à partir des sulfochlorures selon l'invention pour lesquels le pourcentage de la cariation du temps de sommeil varie d'environ 0% à environ 50% sont des potentialisateurs d'activité voisins des composés de référence sultopride et tiapride, utilisés.

Les composés obtenus à partir des sulfochlorures selon l'invention pour lesquels le pourcentage de la variation du temps de sommeil est inférieur à 0% sont des antagonistes et peuvent être utilisés comme antidépresseurs.

On note que le sulpride utilisé à titre de produit de référence se révèle être tantôt potentialisateur, tantôt antagoniste ce qui introduit son double profil de neuroleptique et d'antidépresseur.

**Revendications pour les Etats contractants BE, CH, DE, GB, IT, LI, LU, NL, SE**

1. Sulfohalogénures de formule générale (I):

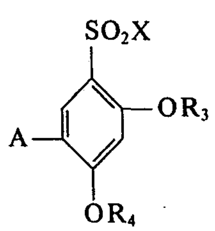

(I)

caractérisés en ce que X représente un halogène, notamment le brome, de préférence le chlore, $R_3$ et $R_4$ représentent indépendamment l'un de l'autre, un radical alcoyle inférieur présentant de 1 à 4 atomes de carbone, A représente un halogène, $NO_2$, $CF_3$, un radical alcoxy ayant de 1 à 4 atomes de carbone, un radical alcoylsulfonyle ayant de 1 à 4 atomes de carbone.

2. Sulfochlorure selon la revendication 1, de formule (II) suivante:

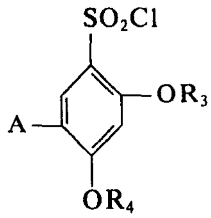

(II)

dans laquelle A, $R_3$ et $R_4$ ont les significations ci-dessus indiquées à la revendication 1.

3. Sulfochlorure selon la revendication 1, de formule (III) suivante:

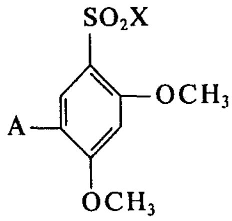

(III)

dans laquelle A et X ont les significations indiquées à la revendication 1.

4. Sulfochlorure selon l'une quelconque des revendications 1 à 4, de formule générale (IV):

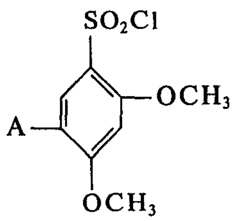

(IV)

dans laquelle A a la significations donnée à la revendication 1.

5. Sulfochlorure selon l'une quelconque des revendications 1 à 4, caractérisé en ce que A représente Cl, Br, $OCH_3$, $SO_2CH_3$, $SO_2C_2H_5$, $SO_2nC_3H_7$, $SO_2iC_3H_7$.

6. Procédé de préparation des sulfochlorures selon l'une quelconque des revendications 1 à 5, caractérisé en ce qu'on a recours à l'arylamine de formule (V):

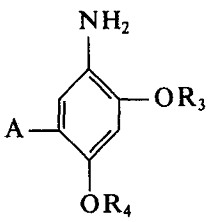

(V)

dans laquelle A, $R_3$ et $R_4$ ont la signification indiquée à la revendication 1 et à partir de laquelle:

a) on forme le sel de diazonium de formule (VI):

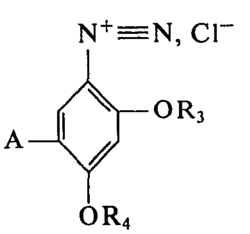

(VI)

notamment par action de l'acide chlorhydrique sur l'amine;

b) on fait ensuite réagir le sel de diazonium obtenu en solution avec de l'anhydride sulfureux, de préférence en présence d'acide acétique et d'un catalyseur, notamment à base de cuivre.

7. Procédé de préparation selon la revendication 6, caractérisé en ce que l'arylamine est choisie dans le groupe constitué par:

- la chloro-5 diméthoxy-2,4 aniline,
- la bromo-5 diméthoxy-2,4 aniline,
- la triméthoxy-2,4,5 aniline,
- la diméthoxy-2,4 méthylsulfonyl-5 aniline,
- la diméthoxy-2,4 éthylsulfonyl-5 aniline,
- la diméthoxy-2,4 propylsulfonyl-5 aniline,
- la diméthoxy-2,4 isopropylsulfonyl-5 aniline.

8. Procédé de préparation des sulfochlorures selon l'une quelconque des revendications 1 à 5, caractérisé en ce que sur le benzène substitué de formule (VII):

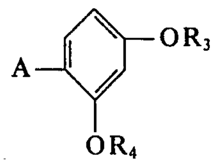

(VII)

dans laquelle $R_3$, $R_4$ et A ont les significations indiquées à la revendication 1, on fait réagir de l'acide sulfurique;

- on transforme éventuellement l'acide sulfonique obtenu de formule (VIII):

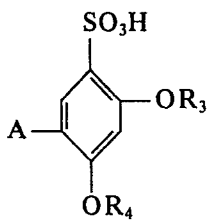

(VIII)

par action d'une base telle que l'hydroxyde de sodium, l'hydroxyde de potassium ou la pyridine, en sel de formule:

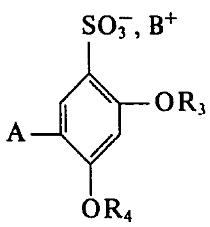

(IX)

dans laquelle $B^+$ représente un métal, notamment alcalin ou alcalino-terreux et A a la signification indiquée à la revendication 1;

- puis on soumet le composé de formule (VIII) ou (IX) à l'action d'un agent chlorant tel que le chlorure de sulfuryle, le pentachlorure de phosphore ou l'oxychlorure de phosphore.

9. Procédé de préparation des sulfochlorures selon l'une quelconque des revendications 1 à 5, caractérisé en ce que l'on fait réagir le chlorure d'acide sulfonique sur le composé de formule (VII):

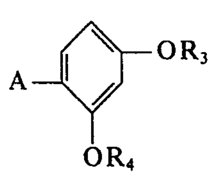

(VII)

dans laquelle A, $R_3$ et $R_4$ ont les significations indiquées à la revendication 1.

10. Application des sulfochlorures selon l'une quelconque des revendications 1 à 5, à la fabrication des composés de formule:

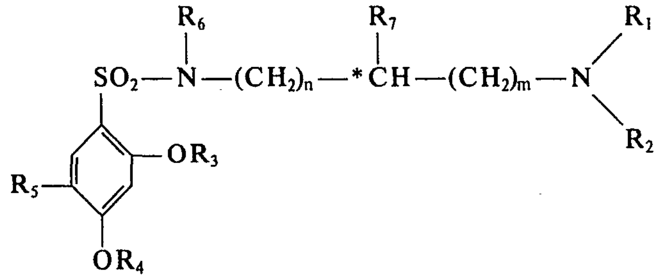

(X)

dans laquelle:

n et m indépendamment l'un de l'autre prennent les valeurs de 0 à 4;

$R_1$ et $R_2$ représentent indépendamment l'un de l'autre, des atomes d'hydrogène, des groupes alcoyle linéaires ou ramifiés ayant de 1 à 4 atomes de carbone, ou formant conjointement avec l'atome d'azote un hétérocycle azoté à 5 ou 6 chaînons, notamment un groupe pyrrolidino, morpholino, pipérazinyle, pyrrole ou pipéridino, substitués ou non par des radicaux alcoyle linéaires ou ramifiés ayant de 1 à 4 atomes de carbone;

$R_3$ et $R_4$ ont les mêmes significations que celles indiquées dans la définition de la formule (I) c'est-à-dire représentent chacun, indépendamment l'un de l'autre, un radical alcoyle inférieur présentant de 1 à 4 atomes de carbone;

$R_5$ a la même signification que celle de A indiquée dans la définition de la formule (I), c'est-à-dire représente un halogène, le groupe $NO_2$, $CF_3$, un radical alcoxy ayant de 1 à 4 atomes de carbone, ou un radical alcoylsulfonyle ayant de 1 à 4 atomes de carbone et peut également représenter le groupe $NH_2$;

$R_6$ et $R_7$ représentent indépendamment l'un de l'autre un atome d'hydrogène, un radical alcoyle de 1 à 6 atomes de carbone ou un radical cycloalcoyle de 3 à 6 atomes de carbone;

ou à la fabrication de leurs sels organiques ou minéraux physiologiquement acceptables, obtenus par action du sulfochlorure de formule (II) sur l'amine de formule:

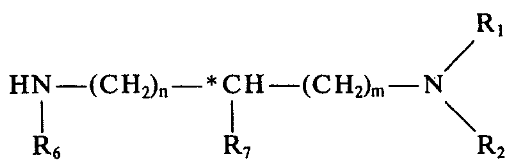

(XIII)

conduisant au chlorhydrate du composé de formule (X), le chlorhydrate obtenu étant éventuellement ensuite désalifié pour l'obtention de la base, et la transformation en sel différent du chlorhydrate pouvant se faire à partir du chlorhydrate du composé de formule (X) ou bien à partir du composé de formule (X).

11. Application selon la revendication 10, des sulfochlorures selon l'une quelconque des revendications 1 à 3, caractérisée en ce que lorsque A représente le groupe $NO_2$, celui-ci peut être réduit par hydrogénation catalytique en $NH_2$, la réduction pouvant avoir lieu sur le composé de formule (X), ou bien sur l'un de ses sels organiques ou minéraux physiologiquement acceptables.

**Revendications pour l'Etat contractant AT**

1. Procédé pour préparer des sulfohalogénures de formule générale (I):

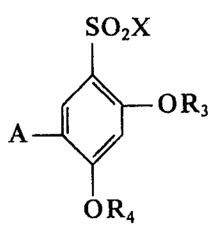

(IV)

dans laquelle X représente un halogène, notamment brome, de préférence chlore, $R_3$ et $R_4$ représentant, indépendamment l'un de l'autre, un radical alcoyle inférieur ayant de 1 à 4 atomes de carbone, A représente un halogène, $NO_2$, $CF_3$, un radical alcoxy ayant de 1 à 4 atomes de carbone, ou un radical alcoylsulfonyle ayant de 1 à 4 atomes de carbone, en ayant recours à l'arylamine de formule:

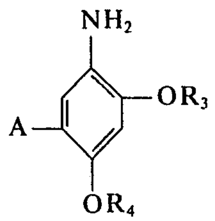

(V)

dans laquelle A, $R_3$ et $R_4$ ont la significations indiquée à la revendication 1 et comprenant:

a) la formation à partir de ladite arylamine d'un sel de diazonium de formule:

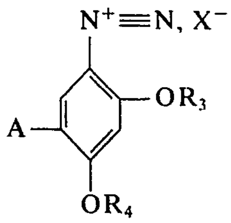

par action de l'acide halogéné sur l'amine;

b) puis la réaction d'un sel de diazonium obtenu en solution avec de l'anhydride sulfureux, de préférence en présence d'acide acétique et d'un catalyseur, notamment à base de cuivre.

2. Procédé pour préparer des sulfohalogénires de formule (I) comme défini dans la revendication 1, caractérisé en ce que sur le benzène substitué de formule (VII):

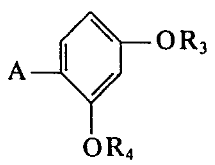

(VII)

dans lequel $R_3$, $R_4$ et A ont les significations indiquées à la revendication 1, on fait réagir de l'acide sulfurique;

– si nécessaire, on transforme l'acide sulfonique de formule (VIII) obtenu:

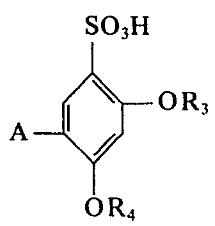

(VIII)

par action d'une base telle que l'hydroxyde de sodium, l'hydroxyde de potassium ou la pyridine, en un sel de formule:

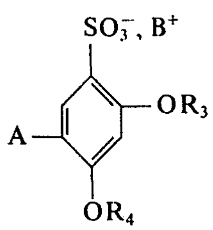

(IX)

dans lequel $B^+$ représente un métal, notamment alcalin ou alcalino-terreux et A a la signification indiquée dans la revendication 1;

– et on soumet le composé de formule (VIII) ou (IX) à l'action d'un agent halogénant.

3. Procédé pour préparer des sulfohalogénures de formule (I) comme défini dans la revendication 1, caractérisé en ce que l'on fait réagir l'halogénure de l'acide sulfonique avec le composé de formule (VII):

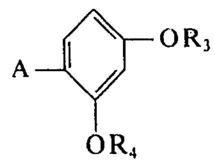

(VII)

dans lequel A, $R_3$ et $R_4$ ont les significations indiquées à la revendication 1.

4. Procédé selon la revendication 1, 2 ou 3, pour préparer des sulfochlorures de formule suivante:

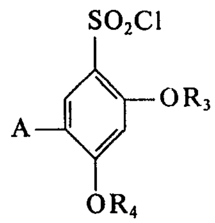

(II)

dans laquelle A, $R_3$ et $R_4$ ont les significations indiquées à la revendication 1.

5. Procédé selon la revendication 1, 2 ou 3, pour préparer des sulfochlorures de formule (III):

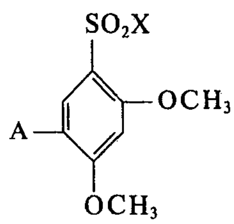

(III)

dans laquelle A et X ont les significations indiquées à la revendication 1.

6. Procédé selon la revendication 1, 2 ou 3, pour préparer les sulfochlorures de formule suivante:

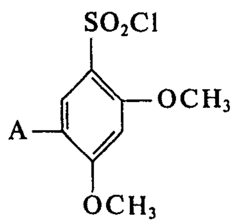

(IV)

dans laquelle A a la signification indiquée dans la revendication 1.

7. Procédé selon la revendication 1, 2 ou 3, pour préparer les sulfochlorures de formule (I), (II), (III) ou (IV) dans lesquelles A représente Cl, Br, $OCH_3$, $SO_2CH_3$, $SO_2C_2H_5$, $SO_2nC_3H_7$, $SO_2iC_3H_7$.

8. Procédé selon la revendication 1, dans lequel l'arylamine est choisie parmi le groupe constitué par:

- 5-chloro-2,4-diméthoxy aniline,
- 5-bromo-2,4-diméthoxy aniline,
- 2,4,5-triméthoxy aniline,
- 2,4-diméthoxy 5-méthylsulfonyl aniline,
- 2,4-diméthoxy 5-éthylsulfonyl aniline,
- 2,4-diméthoxy 5-propylsulfonyl aniline,
- 2,4-diméthoxy 5-isopropylsulfonyl aniline.

**Patentansprüche für die Vertragsstaaten: BE, CH, DE, GB, IT, LI, LU, NL, SE**

1. Sulfonylhalogenide der allgemeinen Formel (I):

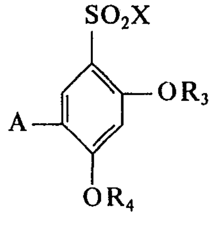

(I)

dadurch gekennzeichnet, daß X ein Halogen, insbesondere Brom, vorzugsweise Chlor bedeutet, $R_3$ und $R_4$ unabhängig voneinander einen niedrigen Alkylrest mit 1 bis 4 Kohlenstoffatomen bedeuten und A ein Halogen, $NO_2$, $CF_3$, einen Alkoxyrest mit 1 bis 4 Kohlenstoffatomen oder einen Alkylsulfonylrest mit 1 bis 4 Kohlenstoffatomen bedeutet.

2. Sulfonylchlorid nach Anspruch 1, der folgenden Formel (II):

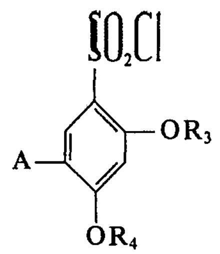

(II)

in welcher A, $R_3$ und $R_4$ die oben im Anspruch 1 angegebenen Bedeutungen haben.

3. Sulfonylchlorid nach Anspruch 1, der folgenden Formel (III):

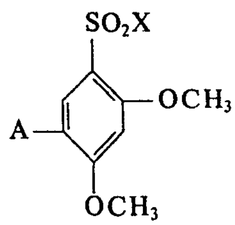

(III)

in welcher A und X die im Anspruch 1 angegebenen Bedeutungen haben.

4. Sulfonylchlorid nach einem der Ansprüche 1 bis 4, der allgemeinen Formel (IV):

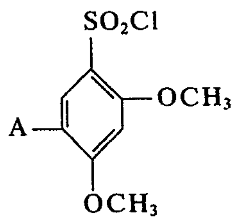

(IV)

in welcher A die im Anspruch 1 angegebene Bedeutung hat.

5. Sulfonylchlorid nach einem der Ansprüche 1 bis 4, dadurch gekennzeichnet, daß A Cl, Br, $OCH_3$, $SO_2CH_3$, $SO_2C_2H_5$, $So_2nC_3H_7$ oder $So_2iC_3H_7$ bedeutet.

6. Verfahren zur Herstellung der Sulfonylchloride nach einem der Ansprüche 1 bis 5, dadurch gekennzeichnet, daß man auf ein Arylamin der Formel (V):

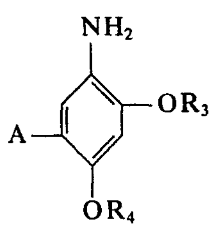

(V)

in welcher A, $R_3$ und $R_4$ die im Anspruch 1 angegebene Bedeutung haben, zurückgreift und ausgehend von diesem:

a) das Diazoniumsalz der Formel (VI):

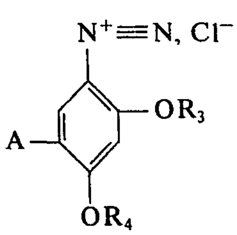

(VI)

insbesondere unter Einwirkung von Chlorwasserstoffsäure auf das Amin, bildet;

b) anschließend das erhaltene Diazoniumsalz in Lösung mit dem Anhydrid der schwefeligen Säure,

vorzugsweise in Gegenwart von Essigsäure und eines Katalysators, insbesondere auf Basis von Kupfer, umsetzt.

7. Herstellungsverfahren nach Anspruch 6, dadurch gekennzeichnet, daß man das Arylamin aus der aus

- Chlor-5-dimethoxy-2,4-anilin,
- Brom-5-dimethoxy-2,4-anilin,
- Trimethoxy-2,4,5-anilin,
- Dimethoxy-2,4-methylsulfonyl-5-anilin,
- Dimethoxy-2,4-ethylsulfonyl-5-anilin,
- Dimethoxy-2,4-propylsulfonyl-5-anilin und
- Dimethoxy-2,4-isopropylsulfonyl-5-anilin

bestehenden Gruppe auswählt.

8. Verfahren zur Herstellung der Sulfonylchloride nach einem der Ansprüche 1 bis 5, dadurch gekennzeichnet, daß man auf ein substituiertes Benzol der Formel (VII):

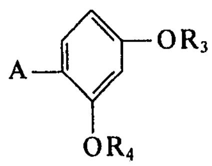

(VII)

in welcher $R_3$, $R_4$ und A die im Anspruch 1 angegebenen Bedeutungen haben, Schwefelsäure einwirken läßt;

- gegebenenfalls die erhaltene Sulfonsäure der Formel (VIII):

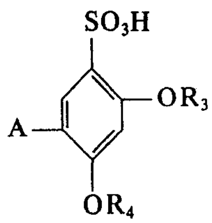

(VIII)

durch Einwirkung einer Base, wie Natriumhydroxid, Kaliumhydroxid oder Pyridin, in ein Salz der Formel:

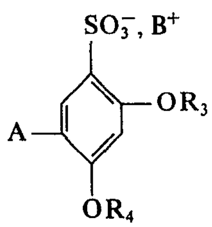

(IX)

in welcher $B^+$ ein Metall, insbesondere Alkalimetall oder Erdalkalimetall bedeutet und A die im Anspruch 1 angegebene Bedeutung hat, umgewandelt;

- und dann die Verbindung der Formel (VIII) oder (IX) der Einwirkung eines Chlorierungsmittels, wie Sulfurylchlorid, Phosphorpentachlorid oder Phosphoroxychlorid, unterwirft.

9. Verfahren zur Herstellung der Sulfonylchloride nach einem der Ansprüche 1 bis 5, dadurch gekennzeichnet, daß man Chlorsulfonsäure mit einer Verbindung der Formel (VII):

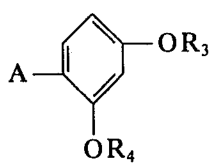

(VII)

in welcher A, $R_3$ und $R_4$ die im Anspruch 1 angegebenen Bedeutungen haben, umsetzt.

10. Anwendung der Sulfonylchloride nach einem der Ansprüche 1 bis 5 bei der Erzeugung von Verbindungen der Formel:

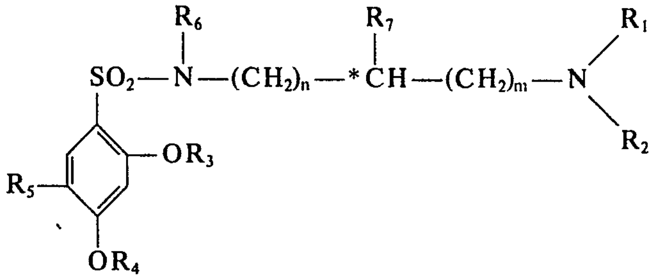

(X)

in welcher:

n und m unabhängig voneinander einen Wert von 0 bis 4 haben;

$R_1$ und $R_2$ unabhängig voneinander Wasserstoffatome oder gerade oder verzweigte Alkylgruppen mit 1 bis 4 Kohlenstoffatomen bedeuten, oder gemeinsam mit dem Stickstoffatom einen stickstoffhaltigen, 5- oder 6gliedrigen Heterocyclus, insbesondere eine Pyrrolidino-, Morpholino-, Piperazinyl-, Pyrrol- oder Piperidinogruppe, die gegebenenfalls durch gerade oder verzweigte Alkylreste mit 1 bis 4 Kohlenstoffatomen substituiert ist, bilden;

$R_3$ und $R_4$ die gleichen Bedeutungen haben wie sie bei der Definition der Formel (I) angegeben sind, d. h. jeweils unabhängig voneinander einen niedrigen Alkylrest mit 1 bis 4 Kohlenstoffatomen bedeuten;

$R_5$ die gleiche Bedeutung wie für A in der Definition der Formel (I) angegeben hat, d. h. ein Halogen, die $NO_2$- oder $CF_3$-Gruppe, einen Alkoxyrest mit 1 bis 4 Kohlenstoffatomen oder einen Alkylsulfonylrest mit 1 bis 4 Kohlenstoffatomen bedeutet, und auch die Gruppe $NH_2$ bedeuten kann; und

$R_6$ und $R_7$ unabhängig voneinander ein Wasserstoffatom, einen Alkylrest mit 1 bis 6 Kohlenstoffatomen oder einen Cycloalkylrest mit 3 bis 6 Kohlenstoffatomen bedeuten;

oder zur Erzeugung ihrer physiologisch annehmbaren organischen oder anorganischen Salze, die durch Einwirkung des Sulfonylchlorids der Formel (II) auf das Amin der Formel:

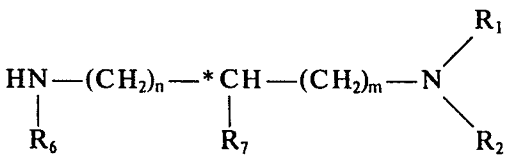

(XIII)

die zum Hydrochlorid der Verbindung der Formel (X) führt, erhalten werden, wobei das erhaltene Hydrochlorid gegebenenfalls anschließend unter Freisetzung der Base behandelt wird, und wobei die Umwandlung in ein vom Hydrochlorid verschiedenes Salz entweder ausgehend vom Hydrochlorid der Verbindung der Formel (X) oder auch ausgehend von der Verbindung der Formel (X) erfolgen kann.

11. Anwendung der Sulfonylchloride nach einem der Ansprüche 1 bis 3 gemäß Anspruch 10, dadurch gekennzeichnet, daß, falls A die $NO_2$-Gruppe bedeutet, diese durch katalytische Hydrierung zu $NH_2$ reduziert werden kann, wobei der Reduktion entweder die Verbindung der Formel (X) oder auch eines ihrer physiologisch annehmbaren, organischen oder anorganischen Salze unterworfen werden kann.

**Patentansprüche für den Vertragsstaat: AT**

1. Verfahren zur Herstellung von Sulfonylhalogeniden der allgemeinen Formel (I):

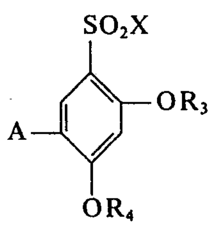

(I)

in welcher X ein Halogen, insbesondere Brom, vorzugsweise Chlor bedeutet, $R_3$ und $R_4$ unabhängig voneinander einen niedrigen Alkylrest mit 1 bis 4 Kohlenstoffatomen bedeuten und A ein Halogen, $NO_2$, $CF_3$, einen Alkoxyrest mit 1 bis 4 Kohlenstoffatomen oder einen Alkylsulfonylrest mit 1 bis 4 Kohlenstoffatomen bedeutet, zurückgreifend auf ein Arylamin der Formel:

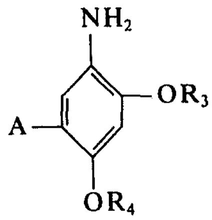

(V)

in welcher A, $R_3$ und $R_4$ die im Anspruch 1 angegebene Bedeutung haben, und umfassend

a) ausgehend von dem genannten Arylamin, die Bildung eines Diazoniumsalzes der Formel:

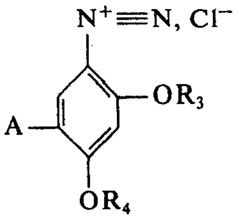

unter Einwirkung von Halogenwasserstoffsäure auf das Amin,

b) anschließend die Umsetzung eines erhaltenen Diazoniumsalzes in Lösung mit dem Anhydrid der schwefeligen Säure, vorzugsweise in Gegenwart von Essigsäure und einem Katalysator, insbesondere auf Basis von Kupfer.

2. Verfahren zur Herstellung von Sulfonylhalogeniden der Formel (I) wie im Anspruch 1 definiert, dadurch gekennzeichnet, daß man auf ein substituiertes Benzol der Formel (VII):

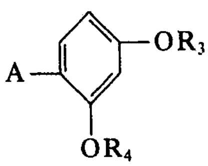

(VII)

in welcher $R_3$, $R_4$ und A die im Anspruch 1 angegebenen Bedeutungen haben, Schwefelsäure einwirken läßt;

– erforderlichenfalls die erhaltene Sulfonsäure der Formel (VIII):

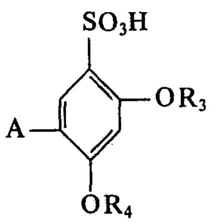

(VIII)

durch Einwirkung einer Base, wie Natriumhydroxid, Kaliumhydroxid oder Pyridin, in ein Salz der Formel:

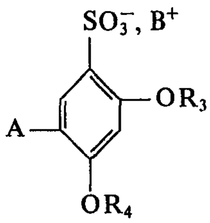

(IX)

in welcher $B^+$ ein Metall, insbesondere Alkalimetall oder Erdalkalimetall bedeutet und A die im Anspruch 1 angegebene Bedeutung hat, umwandelt;

– und dann die Verbindung der Formel (VIII) oder (IX) der Einwirkung eines Halogenierungsmittels unterwirft.

3. Verfahren zur Herstellung der Sulfonylhalogenide der Formel (I) wie im Anspruch 1 definiert, dadurch gekennzeichnet, daß man Halogensulfonsäure mit einer Verbindung der Formel (VII):

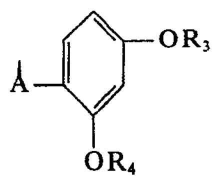

(VII)

in welcher A, $R_3$ und $R_4$ die im Anspruch 1 angegebenen Bedeutungen haben, umsetzt.

4. Verfahren nach Anspruch 1, 2 oder 3 zur Herstellung der Sulfonylchloride folgender Formel:

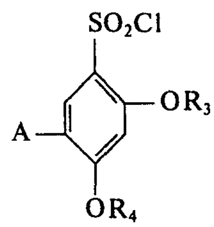

(II)

in welcher A, $R_3$ und $R_4$ die im Anspruch 1 angegebenen Bedeutungen haben.

5. Verfahren nach Anspruch 1, 2 oder 3 zur Herstellung der Sulfonylchloride der Formel (III):

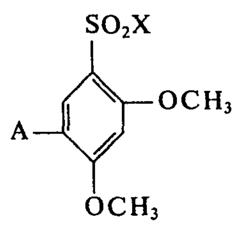

(III)

worin A und X die im Anspruch 1 angegebenen Bedeutungen haben.

6. Verfahren nach Anspruch 1, 2 oder 3 zur Herstellung der Sulfonylchloride der folgenden Formel:

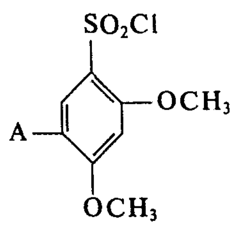

(IV)

in welcher A die im Anspruch 1 angegebene Bedeutung hat.

7. Verfahren nach Anspruch 1, 2 oder 3 zur Herstellung der Sulfonylchloride der Formeln (I), (II), (III) oder (IV), in welchen A Cl, Br, $OCH_3$, $SO_2CH_3$, $SO_2C_2H_5$, $SO_2nC_3H_7$ oder $SO_2iC_3H_7$ bedeutet.

8. Verfahren nach Anspruch 1, in welchem das Arylamin aus der aus:

- 5-Chlor-2,4-dimethoxyanilin,
- 5-Brom-2,4-dimethoxyanilin,
- 2,4,5-Trimethoxyanilin,
- 2,4-Dimethoxy-5-methylsulfonylanilin,
- 2,4-Dimethoxy-5-ethylsulfonylanilin,
- 2,4-Dimethoxy-5-propylsulfonylanilin und
- 2,4-Dimethoxy-5-isopropylsulfonylanilin

bestehenden Gruppe ausgewählt ist.

**Claims for the Contracting States: BE, CH, DE, GB, IT, LI, LU, NL, SE**

1. Sulfohalides of the general formula (I):

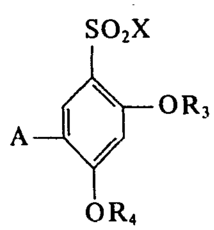

(I)

wherein X represents a halogen, particulary bromine, preferably chlorine, $R_3$ and $R_4$ representing independently of one another, a lower alkyl radical having from 1 to 4 carbon atoms, A represents a halogen, $NO_2$, $CF_3$, an alkoxy radical having from 1 to 4 carbon atoms, or an alkyl sulfonyl radical having from 1 to 4 carbon atoms.

2. Sulfochloride according to claim 1, of the following formula (II):

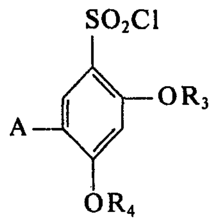

(II)

in which A, $R_3$ and $R_4$ have the meanings indicated above according to claim 1.

3. Sulfochloride according to claim 1, of the following formula (III):

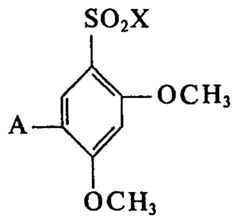

(III)

in which A and X have the meanings indicated in claim 1.

4. Sulfochloride according to any preceding claim, of general formula (IV):

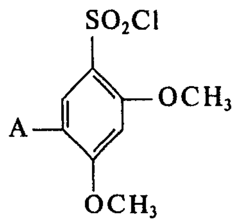

(IV)

in which A has the meaning given in claim 1.

5. Sulfochloride according to any preceding claim, wherein A represents Cl, Br, $OCH_3$, $SO_2CH_3$, $SO_2C_2H_5$, $SO_2nC_3H_7$, $SO_2iC_3H_7$.

6. Process for preparing sulfochlorides according to any preceding claim, comprising the utilization of an arylamine of the formula (V):

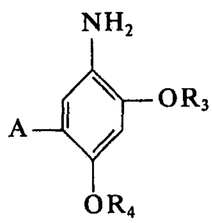

(V)

in which A, $R_3$ and $R_4$ have the meaning indicated in claim 1 and comprising:

a) forming from said arylamine the diazonium salt of formula (VI):

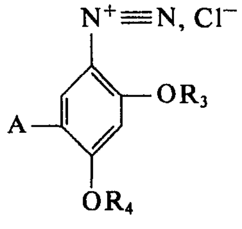

(VI)

particularly by the action of hydrochloric acid on the amine;

b) then reacting the diazonium salt obtained in solution with sulfur dioxide, preferably in the presence of acetic acid and a catalyst, particularly based on copper.

7. Process according to claim 6, wherein the arylamine is selected from the group constituted by:

– 5-chloro 2,4-dimethoxy aniline,
– 5-bromo 2,4-dimethoxy aniline,
– 2,4,5-trimethoxy aniline,
– 2,4-dimethoxy 5-methylsulfonyl aniline,
– 2,4-dimethoxy 5-ethylsulfonyl aniline,
– 2,4-dimethoxy 5-propylsulfonyl aniline,
– 2,4-dimethoxy 5-isopropylsulfonyl aniline.

8. Process for the preparation of sulfochlorides according to any one of claims 1 to 5, comprising reacting sulfuric acid with the substituted benzene of formula (VII):

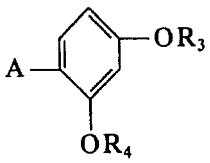

(VII)

in which $R_3$, $R_4$ and A have the meanings indicated in claim 1:

– if necessary, converting the sulfonic acid of formula (VIII) obtained

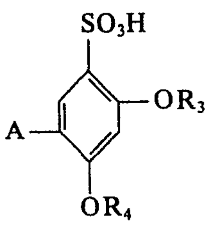

(VIII)

by the action of a base such as sodium hydroxide, potassium hydroxide or pyridine, into a salt of the formula:

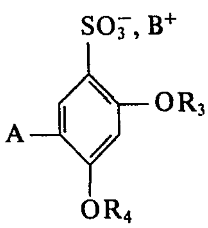

(IX)

in which $B^+$ represents a metal, particularly an alkali or alkaline earth metal and A has the meaning indicated in claim 1;
– and then subjecting the compound of formula (VIII) or (IX) to the action of a chlorinating agent such as sulfuryl chloride, phosphorus pentachloride or phosphorus oxychloride.

9. Process for preparing sulfochlorides according to any one of claims 1 to 5, comprising reacting the sulfonic acid chloride with the compound of formula (VII):

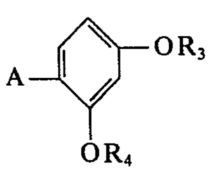

(VII)

in which A, $R_3$ and $R_4$ have the meanings indicated in claim 1.

10. Use of sulfochlorides according to any one of claims 1 to 5, in the preparation of compounds of the formula:

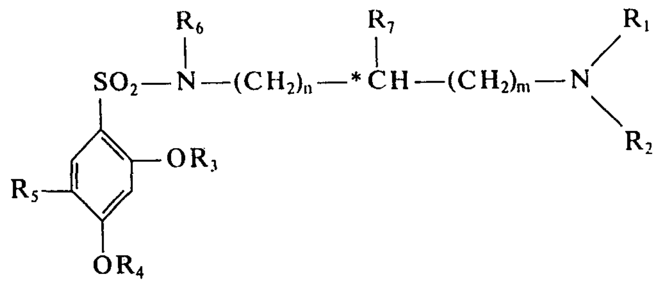

(X)

in which:

n and m independently of one another have values from 1 to 4;

$R_1$ and $R_2$ represent independently of one another, hydrogen atoms, linear or branched alkyl groups having from 1 to 4 carbon atoms, or forming conjointly with the nitrogen atom a nitrogen heterocyclic group with 5 or 6 links, particularly a pyrrolidino, morpholidino, piperazinyl, pyrrole or piperidino group substituted or not by linear or branched alkyl radicale having from 1 to 4 carbon atoms;

$R_3$ and $R_4$ have the same meanings as those indicated in the definition of formula (I), that is to say each represents, independently of one another, a lower alkyl radical having from 1 to 4 carbon atoms;

$R_5$ has the same meaning as that of A indicated in the definition of formula (I), that is to say represents a halogen, the $NO_2$ or $CF_3$ group, and alkoxy radical having from 1 to 4 carbon atoms, or an alkylsulfonyl radical having from 1 to 4 carbon atoms and can also represent the $NH_2$ group;

$R_6$ and $R_7$ may represent independently of one another, a hydrogen atom, an alkyl radical of 1 to 6 carbon atoms or a cycloalkyl radical of 3 to 6 carbon atoms;

or the manufacture of their physiologically acceptable organic or inorganic salts, obtained by the action of the sulfochloride of formula (II) on the amine of formula:

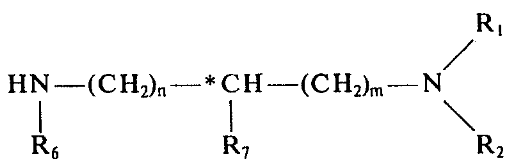

(XIII)

resulting in the hydrochloride of the compound of formula (X), the hydrochloride obtained being then, if desired, desalified to obtain the base, and conversion into a different salt of the hydrochloride being done from the hydrochloride of the compound of formula (X) or from the compound of formula (X).

11. Use according to claim 10, of the sulfochlorides according to any one of claims 1 to 3, wherein when A represents the $NO_2$ group, the latter can be reduced by catalytic hydrogenation into $NH_2$, the reduction being able to take place on the compound of formula (X), or on one of its physiologically acceptable organic or inorganic salts.

**Claims for the Contracting State: AT**

1. Process for preparing sulfohalides of the general formula:

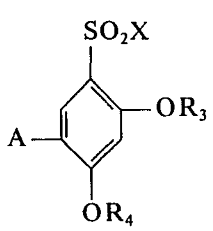

(I)

in which X represents a halogen, particularly bromine, preferably chlorine, $R_3$ and $R_4$ representing independently from each other a lower alkyl radical having from 1 to 4 carbon atoms, A represents halogen, $NO_2$, $CF_3$, an alkoxy radical having from 1 to 4 carbon atoms or an alkylsulfonyl radical having from 1 to 4 carbon atoms, by resorting to an arylamine of formula:

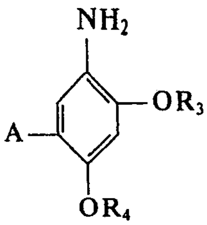

(V)

in which A, $R_3$ and $R_4$ have the meaning mentioned in claim 1 and comprising:

a) forming from said arylamine, a diazonium salt of formula:

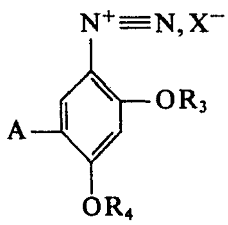

by action of a halogenated acid on the amine;

b) then reacting a diazonium salt obtained in solution with sulfur dioxide, preferably in the presence of acetic acid and of a catalyst, particularly based on copper.

2. Process for preparing sulfohalides of formula (I) as defined in claim 1, characterized by the fact that sulfuric acid is reacted on the substituted benzene of formula (VII):

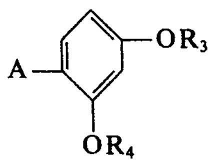

(VII)

in which $R_3$ and $R_4$ and A have the mentioned meaning of claim 1:

– if necessary, the sulfonic acid of formula (VIII) obtained is transformed:

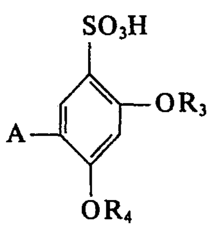

(VIII)

by action of a basis such as sodium hydroxide, potassium hydroxide or pyridine into a salt of formula:

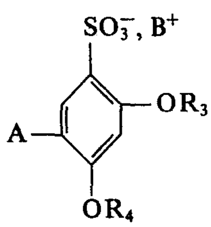

(IX)

in which $B^+$ represents a metal, particularly an alkali or alkaline earth metal and A has the above mentioned meaning according to claim 1;

– and the compound of formula (VIII) or (IX) is submitted to the action of a halogenating agent.

3. Process for preparing sulfohalides of formula as defined in claim 1, characterized by the fact that a sulfonic acid halide is reacted with the compound of formula (VII):

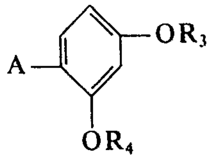

(VII)

in which A, $R_3$ and $R_4$ have the meaning mentioned in claim 1.

4. Process according to claims 1, 2 or 3 for preparing sulfochlorides of the following formula:

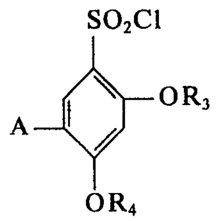

(II)

in which A, $R_3$ and $R_4$ have the meaning mentioned in claim 1.

5. Process according to claims 1, 2 or 3 for preparing sulfochlorides of the following formula (III):

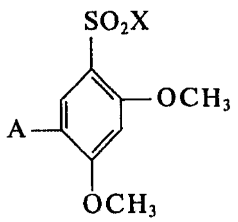

(III)

in which A and X have the meaning mentioned in claim 1.

6. Process according to claims 1, 2 or 3 for preparing sulfochlorides of the following formula:

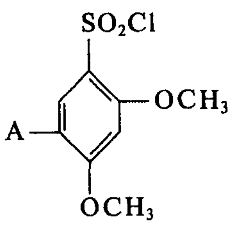

(IV)

in which A has the meaning mentioned in claim 1.

7. Process according to claims 1, 2 or 3 for preparing sulfochlorides of the following formulae (I), (II), (III) or (IV) in which A represents Cl, Br, $OCH_3$, $SO_2CH_3$, $SO_2C_2H_5$, $SO_2nC_3H_7$, $SO_2iC_3H_7$.

8. Process according to claim 1, in which the arylamine is chosen from the group constituted by:

- 5-chloro-2,4-dimethoxy aniline,
- 5-bromo-2,4-dimethoxy aniline,
- 2,4,5-trimethoxy aniline,
- 2,4-dimethoxy 5-methylsulfonyl aniline,
- 2,4-dimethoxy 5-ethylsulfonyl aniline,
- 2,4-dimethoxy 5-propylsulfonyl aniline,
- 2,4-dimethoxy 5-isopropylsulfonyl aniline.